# FASCICULE DE BREVET EUROPEEN

(11) **EP 1 066 248 B1**
(45) Date de publication et mention de la délivrance du brevet: **18.06.2003**
(21) Numéro de dépôt: 99909013.7
(22) Date de dépôt: 17.03.1999
(51) Int. Cl.: C07C 251/24, C07C 251/48, C07C 255/50

(54) **DERIVES DE METHYL-BIPHENYLE, LEUR PROCEDE DE PREPARATION ET LEUR UTILISATION**
METHYLBIPHENYL-DERIVATE, VERFAHREN ZU IHRER HERSTELLUNG UND IHRE VERWENDUNG
METHYL-BIPHENYL DERIVATIVES, PREPARATION METHOD AND USE

(30) Priorité: 24.03.1998 FR 9803621
(43) Date de publication de la demande: 10.01.2001
(73) Titulaire: SANOFI-SYNTHELABO, 75013 Paris (FR)
(72) Inventeur: DORMOY, Jean-Robert, F-04200 Sisteron (FR); GOUBET, Dominique, F-34270 Les Matelles (FR); MOREAU, Patrice, F-34980 Saint-Gely-du-Fesc (FR)
(74) Mandataire: Thouret-Lemaitre, Elisabeth
(86) Numéro de dépôt international: FR9900593
(87) Numéro de publication internationale: WO99048861

(56) Documents cités:
- EP-A- 0 566 468

## Description

La présente invention se rapporte, d'une manière générale, à un nouveau dérivé de méthyl-biphényle, à son procédé de préparation et à son utilisation comme intermédiaire de synthèse.

Plus précisément, l'invention a pour objet l'o-tolyl-benzaldoxime de formule : 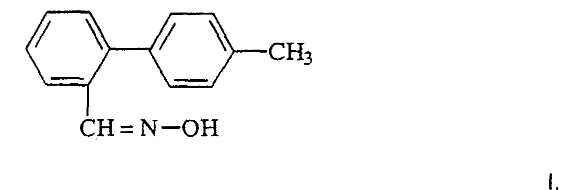 ce composé étant considéré sous la forme de ses isomères individuels ou de mélanges de ceux-ci.

Le dérivé oxime de formule I, ci-après désigné par OTBO, s'est révélé particulièrement utile comme produit intermédiaire notamment pour la préparation du o-(p-tolyl)-benzonitrile, ci-après dénommé OTBN.

Ce dernier peut être lui-même largement utilisé comme intermédiaire particulièrement intéressant puisqu'il constitue l'intermédiaire clé dans la synthèse de nombreux principes actifs de médicaments agissant notamment contre l'hypertension par un mécanisme d'inhibition de l'angiotensine II.

L'OTBN a été décrit pour la première fois dans le brevet EP 253310 et un certain nombre de procédés pour sa synthèse ont été proposés récemment.

Un des procédés qui semble le plus approprié pour la préparation de l'OTBN a été décrit dans le brevet EP 566468. Il consiste en la réaction entre un o-halobenzonitrile avec un halogénure de p-tolylmagnésium en présence d'un sel manganeux, de préférence MnCl₂. Cependant, cette méthode fournit comme sous-produit de réaction de 6,5 à 10% en poids de 4,4'-diméthylbiphényle, ci-après dénommé bis-tolyle, résultant de la condensation de l'halogénure de p-tolylmagnésium sur lui-même.

Dans le cadre de l'invention, on a recherché, en vue de résoudre le problème ci-dessus, la possibilité de préparer l'OTBN par l'intermédiaire d'un de ses précurseurs potentiels en l'occurrence l'o-(p-tolyl)-benzaldoxime.

A cet effet, on a tenté d'appliquer un procédé analogue à celui du brevet EP 253310 en mettant également en oeuvre le bromure de p-tolylmagnésium.

Toutefois, des essais pratiqués au départ de 2-chlorobenzaldoxime et de 3,5 équivalents de bromure de p-tolylmagnésium, la réaction se déroulant en présence de 0,36 équivalent de MnCl₂ dans le tétrahydrofuranne à 90°C et durant 8 heures, n'a pas permis d'observer la réaction de couplage attendue mais la production massive de bis-tolyle.

La recherche d'un procédé de préparation de l'OTBN au départ par exemple de l'oxime correspondante elle-même obtenue de manière avantageuse et exempte des inconvénients cités ci-dessus, reste d'un intérêt incontestable.

Or, on a découvert de manière surprenante que l'o-(p-tolyl)-benzaldoxime peut être obtenue avec excellents rendements et moins de 6% de sous-produit bis-tolyle par réaction de couplage mettant en oeuvre le bromure de p-tolylmagnésium et non pas le 2-chlorobenzaldoxime mais une 2-haiogénobenzaldimine N-substituée de manière à former une o-(p-tolyl)-benzaldimine N-substituée aisément transformable en l'oxime souhaitée.

Selon l'invention, on obtient cette oxime de formule I par réaction d'un sel d'hydroxylamine, avec un dérivé benzaldimine de formule générale : 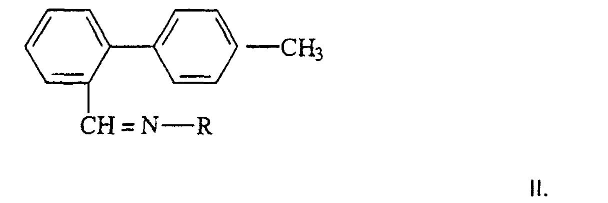 dans laquelle R représente un groupement alkyle, linéaire ou ramifié, en C₃-C₇ ou cycloalkyle en C₃-C₇, ce composé de formule II étant considéré sous forme d'isomères individuels ou de mélange de ceux-ci, ce qui fournit les composés désirés.

Cette réaction se déroule habituellement à une température comprise entre 0°C et 10°C, de préférence entre 0°C et 5°C et dans un solvant aprotique.

Par "solvant aprotique" on entend, dans le cadre de la présente invention, un solvant tel qu'un éther généralement un éther aliphatique ou alicyclique par exemple le tétrahydrofuranne, le méthyl-tertiobutyléther, le dibutyléther ou le dioxane, un hydrocarbure aliphatique ou aromatique tel que le benzène, le toluène ou un xylène ou un hydrocarbure halogéné tel que le dichlorométhane, le dichloroéthane, le chloroforme ou le tétrachloroéthane.

On préfère toutefois utiliser un éther, comme solvant par exemple le tétrahydrofuranne.

Par ailleurs, le sel d'hydroxylamine tel que le chlorhydrate ou de préférence le sulfate, intervient à raison de 1,5 à 2,5 équivalents molaires par équivalent molaire de dérivé de benzaldimine de formule II.

Selon cette méthode, l'OTBO peut être obtenu avec des rendements de l'ordre de 90 à 93% en poids.

Les dérivés de méthyl-biphényle de formule II sont nouveaux et, à ce titre, constituent un autre objet de l'invention qu'ils soient sous forme d'isomères individuels ou de mélange de ceux-ci.

En conséquence, l'invention se rapporte également, en tant que produits intermédiaires nouveaux, aux dérivés de benzaldimine de formule II dans laquelle R représente un groupement alkyle, linéaire ou ramifié, en C₃-C₇ ou un groupement cycloalkyle en C₃-C₇, ces dérivés de benzaldimine étant sous forme d'isomères individuels ou de mélanges de ceux-ci.

Parmi ces composés de formule II, ceux dans lesquels R représente un groupement tertiobutyle ou mieux un groupement cyclohexyle constituent des composés préférés.

Les composés de formule II peuvent être préparés en faisant réagir, en présence d'un dérivé minéral du manganèse, un dérivé benzaldimine de formule générale : 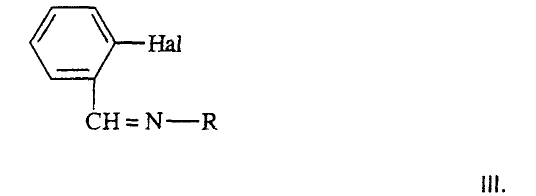 dans laquelle R a la même signification que précédemment et Hal représente un atome d'halogène tel que chlore ou brome, ce composé étant sous forme d'isomères individuels ou de mélanges de ceux-ci, avec un halogénure de p-tolylmagnésium tel que le chlorure ou le bromure de p-tolylmagnésium, ce qui fournit les composés désirés.
Généralement, cette réaction de couplage s'effectue dans un solvant approprié et à une température comprise entre -10°C et la température de reflux, de préférence à la température de reflux du milieu réactionnel.

Comme solvant, on envisage habituellement un composé de type éther tel qu'un éther aliphatique ou alicyclique par exemple le tétrahydrofuranne, le méthyl-tertiobutyléther, le dibutyléther ou le dioxane.

Toutefois, le tétrahydrofuranne constitue un solvant préféré.

En outre, on utilise généralement l'halogénure de p-tolylmagnésium en excès en particulier à raison de 1 à 2 équivalents molaires par équivalent molaire de composé de formule II, habituellement à raison d'environ 1,5 équivalent.

Le dérivé minéral du manganèse intervient dans la réaction à raison de 0,1 à 0,5 équivalent molaire par équivalent molaire de dérivé de benzaldimine de formule II, de préférence de 0,15 à 0,30 équivalent molaire.

Il s'agit généralement d'un sel ou d'un oxyde de manganèse mais plus particulièrement d'un sel manganeux ou de l'oxyde manganeux. Toutefois le sel manganeux correspond de préférence à MnCl₂ ou à MnCl₄Li₂, ce dernier pouvant être formé ***in situ*** par addition de deux équivalents molaires de LiCl et d'un équivalent molaire de MnCl₂.

De cette manière, les composés de formule II peuvent être obtenus avec des rendements d'au moins 85% et avec moins de 6% de dérivé bis-tolyle.

Par exemple, la préparation de l'o-(p-tolyl)-N-cyclohexyl-benzaldimine à partir de 0,4 mole de 2-chloro-N-cyclohexyl-benzaldimine, 0,15 équivalent molaire de MnCl₂ et 1,5 équivalent molaire de chlorure de p-tolylmagnésium dans le tétrahydrofuranne durant 1 heure n'a produit, à côté d'un excellent rendement en OTBN, que 5,5% de bis-tolyle par rapport à l'imine de départ.

Quant aux dérivés benzaldimine de formule III, ceux-ci peuvent être préparés par réaction à une température comprise entre la température ambiante et la température de reflux et dans un solvant aprotique, de préférence un éther entre un 2-chloro- ou 2-bromo-benzaldéhyde et une amine de formule générale :

R - NH₂ IV

dans laquelle R a la même signification que précédemment, ce qui permet d'obtenir les composés désirés.

Cette réaction se déroulant avec formation d'eau, il peut être avantageux de l'entreprendre en présence d'un agent de déshydratation du milieu réactionnel tel que le sulfate de magnésium anhydre.

Comme indiqué précédemment, le dérivé oxime de formule I peut être utilisé pour la préparation de l'OTBN.

En conséquence, l'invention se rapporte à l'o-(p-tolyl)-benzaldoxime en tant qu'intermédiaire pour la synthèse finale de l'OTBN.

Ainsi, on peut obtenir l'OTBN au départ du dérivé oxime de formule I, par exemple en le soumettant à l'action d'un agent déshydratant.

La réaction qui en découle s'effectue habituellement à une température comprise entre la température ambiante et la température de reflux du milieu, et dans un solvant aprotique de préférence un éther tel que le tétrahydrofuranne.

Par "agent déshydratant", on entend dans le cadre de l'invention, un agent capable de transformer la fonction oxime en fonction nitrile tel que par exemple l'acide formique, l'anhydride phosphorique, l'oxychlorure de phosphore, la pyridine ou le dicyclohexylcarbodiimide.

En outre, la réaction de déshydratation se déroule habituellement dans un solvant aprotique de préférence et avantageusement dans un éther tel que par exemple le tétrahydrofuranne et à une température comprise entre la température ambiante et la température de reflux, de préférence à la température de reflux du milieu réactionnel.

Toutefois, cette réaction de déshydratation peut être entreprise en l'absence de solvant, l'agent déshydratant remplissant lui-même la fonction de solvant. Tel est le cas notamment de l'acide formique qui peut être utilisé à la fois comme agent déshydratant et comme solvant dans le cadre de l'invention.

Selon la méthode ci-dessus, on obtient l'OTBN avec des rendements en produit purifié et cristallisé supérieurs à 85%, en général de l'ordre de 90 à 95% à partir de l'OTBO.

L'oxime de formule I et les dérivés de benzaldimine de formule II intervenant dans la synthèse finale de l'OTBN peuvent être utilisés après isolement du milieu réactionnel où ils sont formés.

D'une manière avantageuse et préférée, on prépare cependant l'OTBN dans le milieu même où l'on forme l'OTBO sans isolement de ce dernier.

En conséquence, l'invention se rapporte également à la préparation de l'OTBN à partir d'un dérivé benzaldimine de formule II :
soit :
(a) par réaction de cette imine dans un solvant aprotique, avec un sel d'hydroxylamine à une température comprise entre 0°C et 10°C, de manière à former transitoirement et sans isolement l'oxime de formule I que l'on traite au moyen d'un agent déshydratant à une température comprise entre la température ambiante et la température de reflux ce qui fournit le composé désiré.
soit :
(b) par réaction de cette imine avec l'acide hydroxylamine-O-sulfonique (H₂N-O-SO₃H) dans un milieu biphasique formé d'eau et d'un solvant aprotique et à une température comprise entre la température ambiante et la température de reflux, pour obtenir transitoirement et sans isolement l'oxime de formule I en mélange avec l'OTBN, mélange que l'on traite au moyen d'un agent déshydratant à une température comprise entre la température ambiante et la température de reflux, ce qui fournit le composé désiré.
   Cette méthode, qui se déroule habituellement dans un solvant aprotique, fournit d'abord un mélange transitoire d'OTBN/OTBO, généralement un mélange de 55 à 75% en poids d'OTBN/45 à 25% en poids d'OTBO, ensuite l'OTBN lui-même avec un rendement global supérieur à 90% et moins de 6% de composé bis-tolyle.
soit :
(c) par hydrolyse de cette imine, dans un solvant aprotique, pour obtenir l'o-(p-tolyl)-benzaldéhyde que l'on fait réagir avec un sel d'hydroxylamine à une température comprise entre 0°C et 10°C, ce qui fournit transitoirement et sans isolement l'oxime de formule I que l'on traite, avec un agent déshydratant à une température comprise entre la température ambiante et la température de reflux du milieu, ce qui fournit le composé désiré.
   Suivant des mises en oeuvre alternatives, on peut préparer l'OTBO et par la suite l'OTBN au départ des dérivés imine de formule III sans isolement des produits intermédiaires formés.
   Par exemple, on fait réagir, dans un éther tel que le tétrahydrofuranne, un dérivé imine de formule III en question avec un halogénure de p-tolylmagnésium en présence d'un dérivé minéral du manganèse et, généralement, à une température comprise entre -10°C et la température de reflux, pour former un dérivé benzaldimine de formule II que l'on transforme alors, sans isolement, de son milieu réactionnel en OTBO de formule I puis, en OTBN selon l'une des méthodes (a), (b) ou (c) ci-dessus.

Les Exemples non limitatifs suivants illustrent l'invention.

Dans ces Exemples, les abréviations suivantes ont été utilisées :
- CG :: chromatographie gazeuse
- SM :: spectre de masse
- IR :: spectre infra-rouge
- RMN :: résonance magnétique nucléaire
- OTBCI :: o-tolyl-N-cyclohexyl-benzaldimine
- OTBO:: o-(p-tolyl)-benzaldoxime
- OTBA :: o-(p-tolyl)-benzaldéhyde
- OTBTBI :: o-(p-tolyl)-tertiobutyl-benzaldimine
- t :: temps de rétention.

### PREPARATIONS

### A) 2-Chloro-N-cyclohexyl-benzaldimine

Dans un ballon bicol de 50ml, muni d'un agitateur magnétique et surmonté d'un réfrigérant ascendant, on charge 9,70g (0,0806mole; 1,132 équivalent) de sulfate de magnésium anhydre. On passe un courant d'azote pendant 10 minutes dans le ballon, puis on ajoute 8ml (10,01g; 0,0712 mole; 1 équivalent) de 2-chloro-benzaldéhyde dilués dans 20ml de tétrahydrofuranne.

Sous agitation, on porte le mélange au reflux pendant 10 minutes (température du bain = 90°C). On introduit ensuite 8,15ml (7,07g; 0,0713 mole; 1 équivalent) de cyclohexylamine, goutte à goutte, en 5 minutes de manière à ne pas rompre le reflux que l'on poursuit encore pendant 2 heures.

De cette manière, on obtient une solution de 2-chloro-N-cyclohexyl-benzaldimine CQ/SM : t (2-chloro-N-cyclohexyl-benzaldimine) = 9,92min; m/z(ion,%) = 223 (M⁺/Cl 37,10); 222 (M⁺-H/Cl 37,10); 221 (M⁺/Cl 35,30); 221 (M⁺-H/Cl 35,30)
IR (CCl₄) : ν (cm⁻¹) : 3071 (faible, élongation CH aromatique) ; 2931 et 2856 (fortes, élongation CH alkyle) ; 1636 (forte, élongation CN) ; 1592, 1568, 1470, 1450 et 1440 (médium à fortes, élongation CC aromatique) ; 1383, 1346, 1274 (médium à fortes, déformation dans le plan CH aromatique).

### B) 2-Chloro-N-cyclohexyl-benzaldimine

On dilue 11ml (13,728g ; 0,0977 mole) de 2-chlorobenzaldéhyde dans 50ml de toluène puis on ajoute en une fois 12ml (10,404g ; 0,105 mole ; 1,07 équivalent) de cyclohexylamine, ce qui provoque une réaction exothermique. La température monte de 18°C jusqu'à 38°C. On porte alors le mélange réactionnel au reflux (température du bain = 124°C). La solution devient trouble.

On élimine l'eau formée avec un système Dean-Stark puis après 3 heures de reflux on arrête la réaction. On refroidit le mélange réactionnel à température ambiante et on évapore le toluène à l'aide d'un évaporateur rotatif, ce qui donne 20,22g d'un liquide visqueux brunâtre (0,091 mole soit un rendement de 93,3%) qui cristallise doucement avec le temps.

De cette manière, on obtient le 2-chloro-N-cyclohexyl-benzaldimine.

### C) 2-Chloro-N-tertiobutyl-benzaldimine

Dans un ballon bicol de 50ml, muni d'un agitateur magnétique et d'un réfrigérant ascendant, on ajoute, sous agitation, 11 ml (13,728g ; 0,0977 mole) de 2-chlorobenzaldéhyde à 16ml (11,136g ; 0,152 mole ; 1,56 équivalent) de tertiobutylamine, ce qui donne lieu à une réaction exothermique. La température s'élève de 16°C jusqu'à 37°C. Un voile rouge apparaît alors dans la solution jaunâtre. On ajoute ensuite 15ml de toluène, ce qui trouble la solution.

On porte alors le mélange final à 50°C pendant 2 heures puis à 127°C pendant 1 heure dans un montage de type Dean & Stark de manière à éliminer l'eau et après 30 minutes de reflux on arrête la réaction.

On refroidit la solution à température ambiante et on évapore le toluène à l'évaporateur rotatif.

De cette manière, on obtient 17,99g (0,092 mole) de 2-chloro-N-tertiobutylbenzaldimine sous forme d'un liquide visqueux jaunâtre qui cristallise lentement.
Rendement : 94%
GC/MS : t = 5,85min; m/z (ion,%) = 197 (M⁺/Cl 37,1) ; 196 (M⁺-H/Cl 37,1) ; 195 (M⁺/Cl 35,5) ; 194 (M⁺-H/Cl 35,5)
I.R. (CCl₄) : ν (cm⁻¹) : 3080, 2940 (faibles, élongation CH aromatique) ; 2970 (forte, élongation CH alkyle) ; 1636 (forte, élongation CN) ; 1593, 1568, 1471 et 1441 (médium à fortes, élongation CC aromatique) ; 1372 à 1274 (médium à fortes, déformation dans le plan CH aromatique).

### EXEMPLE 1

### o-(p-Tolyl)-N-cyclohexyl-benzaldimine

Dans un ballon tricol de 250ml contenant 1,34g (0,0106 mole; 0,15 équivalent) de chlorure de manganèse, on filtre sous azote, la solution de 1 équivalent de 2-chloro-N-cyclohexyl-benzaldimine obtenue à la Préparation A et on lave le sulfate de magnésium avec 58,85ml de tétrahydrofuranne anhydre et sous azote. On ajoute alors le filtrat obtenu au filtrat précédent.

Sous agitation magnétique, on porte ensuite pendant 10 minutes au reflux (température du bain = 92°C), la suspension finale ainsi obtenue contenant 0,75 mole de 2-chloro-N-cyclohexyl-benzaldimine et on y ajoute, goutte à goutte, au reflux et en 30 minutes, 1,52 équivalent de chlorure de p-tolylmagnésium.

La suspension devient foncée, passe par une couleur verte éphémère, devient rouge-sang puis termine par le brun foncé. Après coulée du dérivé magnésien, on maintient le reflux pendant 1 heure puis on prélève un échantillon que l'on traite par un mélange eau/glace et extrait à l'éther diéthylique. On analyse alors la phase organique en chromatographie gazeuse, ce qui permet d'observer le composé désiré ainsi que la présence de bis-tolyle, de traces de p-crésol et éventuellement d'OTBA.

De cette manière, on obtient une solution de o-(p-tolyl)-N-cyclohexylbenzaldimine.
CG/SM : t (OTBCI) : 13,47 min ; m/z (ion,%) = 277 (M⁺, 20) ; 276 (M⁺-H, 100) ; 194 (M⁺-cyclohexyle, 70).

En suivant le même procédé que précédemment toutefois au départ de la 2-chloro-N-tertiobutyl-benzaldimine, on a préparé la o-(p-tolyl)-N-tertlobutyl-benzaldimine (Exemple 2)
CG/SM : t (OTBTBI) : 10,87 min ; m/z (ion, %) = 251 (M⁺,5) ; 250 (M⁺-H,10) ; 236 (M⁺-CH₃, 70) ; 194 (M⁺-tertiobutyle, 100) ; 179 (M⁺-tertiobutyl-CH₃, 100).

### EXEMPLE 3

### o-(p-Tolyl)-benzaldoxime

On refroidit le mélange réactionnel obtenu à l'Exemple 1 à température ambiante et sans agitation de façon à décanter les espèces inorganiques qui précipitent (poudre brune), puis on le coule lentement sous agitation dans 150ml d'une solution glacée (0°C à 5°C) de 23,37g (0,1424 mole; 2 équivalents) de sulfate d'hydroxylamine.

On agite alors vigoureusement le mélange biphasique pendant une heure et on laisse revenir à température ambiante.

Sous agitation, on ajoute alors au mélange 3ml (2,65g ; 0,0226 mole ; 2 équivalents par rapport au chlorure de manganèse) de N,N-diéthyléthanolamine.

Après 10 minutes, on arrête l'agitation et on laisse décanter les phases.

On récupère la phase organique supérieure et on extrait la phase aqueuse avec 3 fois 100ml de dichlorométhane (pH de la phase aqueuse = 4). On sèche la phase organique totale sur sulfate de magnésium, filtre puis évapore sous vide à l'aide d'un évaporateur rotatif, ce qui fournit 11,00g de flocons blancs (rendement chimique global : 73% à partir du 2-chlorobenzaldéhyde, la quantité massique de bis-tolyle dans le mélange étant de 4%).

Au solide ainsi obtenu, on ajoute 50ml d'éther de pétrole (fraction 30-40°C) et on agite durant 15 minutes. On filtre le solide blanc formé et on le rince à froid avec 10ml d'éther de pétrole.

De cette manière, on recueille 10,62g de o-(p-tolyl)-benzaldoxime sous forme de flocons blancs ne contenant plus de traces de bis-tolyle.
Rendement global brut : 67,5% à partir du 2-chlorobenzaldéhyde.

L'oxime ainsi obtenue peut être recristallisée (dichlorométhane / éther de pétrole) pour donner des paillettes blanches. Pureté : 100%.
CG/SM : t (OTBO) : 10,97 min ; m/z (ion, %) = 211 (M⁺, 25) ; 210 (M⁺,H, 100) ; 194 (M⁺-OH, 60).
IR (CCl₄) : ν (cm⁻¹) : 3596 (forte, élongation OH libre, solution diluée) ; 3312 (faible, élongation OH associé) ; 3061, 3026 et 2924 (faibles, élongation CH aromatique) ; 1516, 1480, 1447 et 1397 (faibles à médium, élongation CC aromatique) ; 1260, 1200 et 1112 (faibles à médium, déformation dans le plan CH aromatique) : 952 (forte, élongation NO)
¹H RMN : (CDCl₃) δ (ppm) : 2,45 (large s, 3H, CH₃) ; 7,28 (large m, 4H, H8-H9-H11-H12) ; 7,43 (large m, 3H, H4-H5 et CHN) ; 7,95 (large m, 1H, H3) ; 8,22 (large s, 1H, H2) et 9,27 (large, 1H, OH).
¹³C RMN : (CDCl₃) δ (ppm) : 21,22(CH₃) ; 126,20 ; 127,53 ; 129, 15 ; 129,59 ; 129,69 ; 129,84 et 130,37 (CH aromatiques) ; 136,56 ; 137,42 et 142, 36 (C aromatique) et 149, 85 (CH = NOH).

### EXEMPLE 4

### o-(p-Tolyl)-benzaldéhyde

Dans un ballon tricol de 250ml contenant 1,34g (0,0106 mole ; 0,15 équivalent) de chlorure de manganèse, on filtre sous azote, la solution de 1 équivalent de 2-chloro-N-cyclohexyl-benzaldimine obtenue à la Préparation A et on poursuit l'opération comme décrit à l'Exemple 1 notamment par ajout de 1,52 équivalent de chlorure de p-tolytmagnésium,

On stoppe alors la réaction par introduction dans un mélange de 200ml d'eau/glace et on extrait avec 3 fois 100ml de dichlorométhane après filtration sur papier d'un dépôt brun très visqueux. On sèche la phase organique totale sur sulfate de mangésium, filtre puis évapore sous vide, ce qui fournit 16,29g d'un liquide visqueux brunâtre. On absorbe sur silice le liquide obtenu et on place le solide formé en tête d'une colonne de silice préparée avec de l'éther de pétrole (fraction 30-40°C). On élue avec ce solvant jusqu'à ce que la totalité du bis-tolyle soit collectée. On récupère ainsi 0,79g (4,34 mmoles) d'un solide sous forme cristalline. On élue alors avec un mélange dichlorométhane / éther de pétrole 5/95 v/v.

De cette manière, on collecte 10,05g (51,26 mmoles) de o-(p-tolyl)-benzaldéhyde sous forme d'un liquide jaunâtre visqueux.
Rendement : 72%
CG/SM : t (OTBA) : 8,78 min ; m/z (ion, %) = 196 (M⁺, 75) ; 195 (M⁺-H, 50) ; 181 (M⁺-CH₃, 100) ; 167 (M⁺-CHO, 40).
IR (CCl₄) : δ (cm⁻¹) : 3066, 3028, 2924, 2848 et 2751 (faibles, élongation CH aromatique) 1598, 1517, 1476, 1445 et 1392 (faibles à médium, élongation CC aromatique) ; 1256 et 1194 (faibles à médium, déformation dans le plan CH aromatique).
¹H RMN : (CDCl₃) δ (ppm) : 2,44 (s, 3H, CH₃) ; 7,26-7,28 (m, 4H, H8-H9-H11-H12) ; 7,42-7,52 (m, 2H, H4-H5) ; 7,59-7,64 (m, 1H, H3) ; 8,00-8,05 (m, 1H, H2) et 10,00 (s, 1H, CHO).
¹³C RMN : (CDCl₃) δ (ppm) : 21,21 (CH₃) ; 127,56 ; 128,95 ; 129,19 ; 129,87 130,06 ; 130,81 et 133,53 (CH aromatiques) ; 133,80 ; 134,84 ; 138,04 et 146,01 (C aromatique et 192,51 (CHO).

### EXEMPLE 5

### o-(p-Tolyl)-benzaldoxime

A température ambiante, on met en solution, dans 50ml de tétrahydrofuranne les 10,05g de o-(p-tolyl)-benzaldéhyde obtenus à l'Exemple 4 puis on ajoute une solution aqueuse de 16,83g (0,1025 mole ; 2 équivalents) de sulfate d'hydroxylamine. On agite violemment le mélange biphasique à température ambiante pendant 1 heure. Une analyse en chromatographie gazeuse indique le disparition de l'OTBA.

On récupère la phase organique supérieure et on extrait la phase aqueuse avec 3 fois 50ml de dichlorométhane (pH de la phase aqueuse = 1). On réunit les phases organiques, sèche sur sulfate de magnésium, filtre sur verre fritté et évapore sous vide.

De cette manière, on obtient 10,07g (0,0477 mole) de o-(p-tolyl)-benzaldoxime.
Rendement : 93%.

### EXEMPLE 6

### o-(p-Tolyl)-benzonitrile

A 0,54g (2,56 mmoles) d'o-(p-tolyl)-benzaldoxime, on ajoute 5ml d'acide formique. On porte la suspension obtenue au reflux pendant une heure et on maintient à cette température durant une heure supplémentaire (température du bain : 126°C).

A la température interne de 54°C, l'ensemble est dissout.

On refroidit la solution à température ambiante, on la coule dans de l'eau et on extrait avec de l'éther diéthylique. On lave la phase éthérée avec une solution d'hydroxyde de sodium 0,5N jusqu'à basicité des eaux de lavage (pH environ 9) puis avec de l'eau jusqu'à neutralité des eaux de lavage (pH environ 7). On sèche la phase organique sur sulfate de magnésium, filtre et évapore sous vide pour donner 0,45g (2,33 mmoles) d'huile qui durcit avec le temps.

De cette manière, on obtient l'o-(p-tolyl)-benzonitrile avec un rendement de 91%.
Pureté : > 95%.

### EXEMPLES 7 à 9

### o-(p-Tolyl)-benzonitrile

On dissout 1 équivalent molaire de o-(p-tolyl)-N-cyclohexyl-benzaldimine dans un mélange 1/1 de tétrahydrofuranne / eau et on ajoute X équivalents molaires d'acide aminohydroxysulfonique. On maintient ce mélange à une température T durant 1 heure, ce qui fournit un mélange d'OTBO / OTBN. On dilue ce mélange dans le tétrahydrofuranne, puis on ajoute 10 équivalents molaires d'anhydride phosphorique et on laisse réagir durant 1 heure supplémentaire à température ambiante.

La couche d'agent déshydratant devient rose et on récupère l'OTBO en mélange avec du bis-tolyle (< 5% massique). Après lavage de la couche d'anhydride phosphorique avec du tétrahydrofuranne, on évapore le solvant.

Selon les quantiés de départ d'acide aminohydroxysulfonique et la température réactionnelle utilisées, on obtient l'o-(p-tolyl)-benzonitrile avec les rendements
suivants :

| **EX.** | **X** | **T(°C)** | **Mélange d'OTBO/OTBN (% en poids)** | **Rendement en OTBN** |
|---|---|---|---|---|
| 7 | 1,9 | 20 | 55/45 | environ 92% |
| 8 | 2,2 | 65 | 55/45 | " |
| 9 | 3,1 | 90 | 75/25 | " |

### EXEMPLES 10 à 12

### o-(p-Tolyl)-benzonitrile

On dissout 1 équivalent molaire d'o-(p-tolyl)-benzaldoxime dans le solvant choisi et on ajoute l'agent déshydratant. On maintient le milieu à une température T durant H heures. On filtre éventuellement le milieu puis on le coule dans de l'eau et on extrait à l'éther diéthylique.

On lave la phase éthérée avec une solution d'hydroxyde de sodium 0,5N puis avec de l'eau jusqu'à neutralité.

On sèche sur sulfate de magnésium, filtre et évapore sous vide.

Selon les solvants, agent déshydratant, température et durée de réaction utilisés, on obtient l'o-(p-tolyl)-benzonitrile avec les rendements suivants

| **EX.** | **Agent déshydratant** | **Solvant** | **T(°C)** | **H (heure)** | **% d'OTBN** | |
|---|---|---|---|---|---|---|
| | | | | | Brut | Purifié (cristallisé) |
| 10 | Anhydride phosphorique (10 équivalents molaires) | | 20 | 1 | 100 | 92 |
| 11 | DCC* (équivalent molaire) | Chlorure de méthylène | 20 | 24 | 100 | 86 |
| 12 | DCC (1 équivalent molaire) | THF** | 90 | 4 | 100 | 90 |

| | | | | | | |
|---|---|---|---|---|---|---|
| * dicyclohexylcarbodiimide | | | | | | |
| ** tetrahydrofuranne | | | | | | |

## Revendications

1. Dérivés de méthyl-biphényle de formule générale : 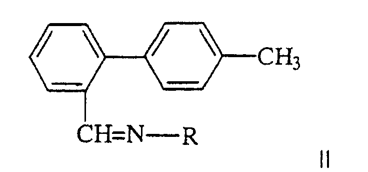 dans laquelle R représente un groupement alkyle, linéaire ou ramifié en C₃-C₇ ou un groupement cycloalkyle en C₃-C₇, ces composés étant considérés sous forme de leurs isomères individuels ou de mélanges de ceux-ci.

2. Dérivé de méthyl-biphényle de formule : 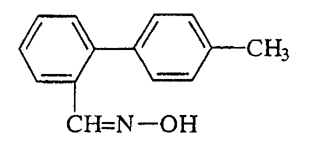 ce composé étant considéré sous la forme de ses isomères individuels ou de mélanges de ceux-ci.

3. Dérivé de méthyl-biphényle selon la Revendication 1, **caractérisé en ce que** R représente cyclohexyle.

4. Dérivé de méthyl-biphényie selon la Revendication 1, **caractérisé en ce que** R représente tertiobutyle.

5. Procédé de préparation du dérivé de méthyl-biphényle selon la Revendication 2, **caractérisé en ce que** l'on fait réagir un sel d'hydroxylamine avec un dérivé benzaldimine selon une des Revendications 1, 3 ou 4, ce qui fournit le composé désiré.

6. Procédé selon la Revendication 5, **caractérisé en ce que** la réaction a lieu à une température comprise entre 0°C et 10°C.

7. Procédé selon la Revendication 5 ou 6, **caractérisé en ce que** la réaction s'effectue dans un solvant aprotique.

8. Procédé selon la Revendication 7, **caractérisé en ce que** le solvant aprotique est un éther aliphatique ou alicyclique, un hydrocarbure aliphatique ou aromatique ou un hydrocarbure halogéné.

9. Procédé selon la Revendication 7 ou 8, **caractérisé en ce que** le solvant aprotique est le tétrahydrofuranne, le méthyl-tertiobutyléther, le dibutyléther ou le dioxane.

10. Procédé de préparation de dérivés de méthyl-biphényle selon une des Revendications 1, 3 ou 4, **caractérisé en ce que** l'on fait réagir, en présence d'un dérivé minéral du manganèse, un dérivé benzaldimine de formule générale: 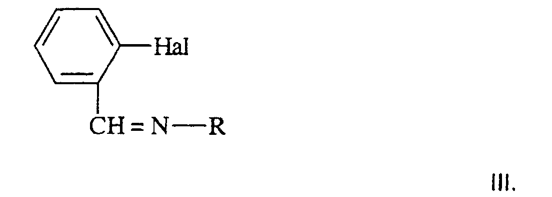 dans laquelle R représente un groupement alkyle, linéaire ou ramifié, en C₃-C₇ ou cycloalkyle en C₃-C₇ et Hal représente un atome d'halogène, ce composé étant sous forme d'isomères individuels ou de mélanges de ceux-ci, avec un halogénure de p-tolylmagnésium, ce qui fournit les composés désirés.

11. Procédé selon la Revendication 10, **caractérisé en ce que** la réaction a lieu à une température comprise entre -10°C et la température de reflux.

12. Procédé selon une des Revendications 10 ou 11, **caractérisé en ce que** la réaction s'effectue dans un éther aliphatique ou alicyclique.

13. Procédé selon une des Revendications 10 ou 11, **caractérisé en ce que** l'éther est le tétrahydrofuranne, le méthyl-tertiobutyléther, le dibutyléther ou le dioxane.

14. Procédé selon une des Revendications 10 à 13, **caractérisé en ce que** l'on utilise de 1 à 2 équivalents molaires de chlorure ou de bromure de p-tolylmagnésium par équivalent molaire de dérivé de benzaldimine de formule III.

15. Procédé selon une des Revendications 10 à 14, **caractérisé en ce que** le dérivé minéral du manganèse est un sel de manganèse ou un oxyde de manganèse.

16. Procédé selon la Revendication 15, **caractérisé en ce que** le sel de manganèse est un sel manganeux et l'oxyde de manganèse est l'oxyde manganeux.

17. Procédé selon la Revendication 16, **caractérisé en ce que** le sel manganeux est MnCl₂ ou MnCl₄ Li₂.

18. Procédé selon une des Revendications 10 à 17, **caractérisé en ce que** le dérivé minéral du manganèse intervient à raison de 0,1 à 0,5 équivalent molaire par équivalent molaire de dérivé de benzaldimine de formule III.

19. Procédé de préparation du o-(p-tolyl)-benzonitrile, **caractérisé en ce que** l'on soumet le dérivé de méthyl-biphényle selon la Revendication 2 à l'action d'un agent déshydratant.

20. Procédé selon la Revendication 19, **caractérisé en ce que** la réaction produite s'effectue à une température comprise entre la température ambiante et la température de reflux du milieu.

21. Procédé selon la Revendication 19 ou 20, **caractérisé en ce que** la réaction se déroule dans un solvant aprotique.

22. Procédé selon la Revendication 21 **caractérisé en ce que** le solvant aprotique est un éther.

23. Procédé selon une des Revendications 19 à 22, **caractérisé en ce que** l'agent déshydratant est l'acide formique, l'anhydride phosphorique, l'oxychlorure de phosphore, la pyridine ou le dicyclohexylcarbodiimide.

24. Procédé de préparation du o-(p-tolyl)-benzonitrile selon la Revendication 19, **caractérisé en ce que** l'on transforme un dérivé benzaldimine de formule II selon la Revendication 1 :
soit :
(a) par réaction avec un sel d'hydroxylamine, dans un solvant aprotique et à une température comprise entre 0°C et 10°C, de manière à former transitoirement et sans isolement l'o-(p-tolyl)-benzaldoxime que l'on traite au moyen d'un agent déshydratant à une température comprise entre la température ambiante et la température de reflux, ce qui fournit le composé désiré,
soit :
(b) par réaction avec l'acide hydroxylamine-O-sulfonique dans un milieu biphasique formé d'eau et d'un solvant aprotique et à une température comprise entre la température ambiante et la température de reflux, pour obtenir transitoirement et sans isolement l'o-(p-tolyl)-benzaldoxime en mélange avec l'o-(p-tolyl)-benzonitrile, mélange que l'on traite au moyen d'un agent déshydratant à une température comprise entre la température ambiante et la température de reflux, ce qui fournit le composé désiré,
soit :
(c) par hydrolyse dans un solvant aprotique, pour obtenir l'o-(p-tolyl)-benzaldéhyde que l'on fait réagir avec un sel d'hydroxylamine à une température comprise entre 0°C et 10°C, ce qui fournit transitoirement et sans isolement l'o-(p-tolyl)-benzaldoxime que l'on traite avec un agent déshydratant à une température comprise entre la température ambiante et la température de reflux du milieu, ce qui fournit le composé désiré.

25. Procédé selon la Revendication 24, **caractérisé en ce que** le dérivé benzaldimine de formule II est obtenu par réaction entre un dérivé benzaldimine de formule générale : 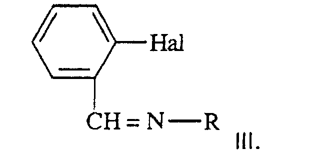 dans laquelle R représente un groupement alkyle, linéaire ou ramifié, en C₃-C₇ ou cycloalkyle en C₃-C₇, ce composé étant sous forme d'isomères individuels ou de mélanges de ceux-ci, avec un halogénure de p-tolylmagnésium en présence d'un dérivé minéral du manganèse, puis transformé sans isolement de son milieu réactionnel.

## Patentansprüche

1. Methyl-biphenyl-Derivate der allgemeinen Formel: 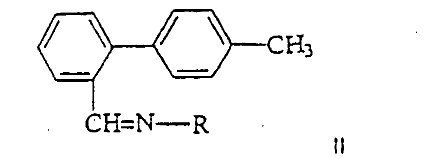 in der R eine geradkettige oder verzweigte C₃-C₇-Alkylgruppe oder eine C₃-C₇-Cycloalkylgruppe bedeutet, wobei diese Verbindungen in Form ihrer einzelnen Isomeren oder von Mischungen davon vorliegen können.

2. Methyl-biphenyl-Derivat der Formel: 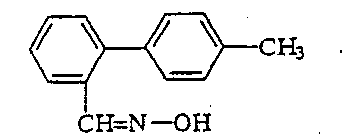 wobei diese Verbindung in Form ihrer einzelnen Isomeren oder ihrer Mischungen vorliegen kann.

3. Methyl-biphenyl-Derivat nach Anspruch 1, **dadurch gekennzeichnet, daß** R Cyclohexyl bedeutet.

4. Methyl-biphenyl-Derivat nach Anspruch 1, **dadurch gekennzeichnet, daß** R tert.-Butyl bedeutet.

5. Verfahren zur Herstellung des Methyl-biphenyl-Derivats nach Anspruch 2, **dadurch gekennzeichnet, daß** man ein Hydroxylaminsalz mit einem Benzaldimin gemäß einem der Ansprüche 1, 3 oder 4 zur Bildung der gewünschten Verbindung umsestzt.

6. Verfahren nach Anspruch 5, **dadurch gekennzeichnet, daß** die Reaktion bei einer Temperatur zwischen 0°C und 10°C durchgeführt wird.

7. Verfahren nach Anspruch 5 oder 6, **dadurch gekennzeichnet, daß** die Reaktion in einem aprotischen Lösungsmittel durchgeführt wird.

8. Verfahren nach Anspruch 7, **dadurch gekennzeichnet, daß** das aprotische Lösungsmittel ein aliphatischer oder alicyclischer Ether, ein aliphatischer oder aromatischer Kohlenwasserstoff oder ein halogenierter Kohlenwasserstoff ist.

9. Verfahren nach Anspruch 7 oder 8, **dadurch gekennzeichnet, daß** das aprotische Lösungsmittel Tetrahydrofuran, Methyl-tert.-butylether, Dibutylether oder Dioxan ist.

10. Verfahren zur Herstellung der Methyl-biphenyl-Derivate nach einem der Ansprüche 1, 3 oder 4, **dadurch gekennzeichnet, daß** man ein Benzaldimin-Derivat der allgemeinen Formel: 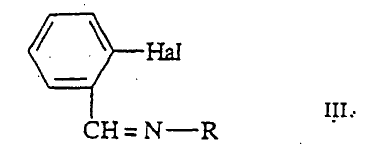 in der R eine geradkettige oder verzweigte C₃-C₇-Alkylgruppe oder eine C₃-C₇-Cycloalkylgruppe und Hal ein Halogenatom bedeuten, wobei diese Verbindung in Form der einzelnen Isomeren oder von Mischungen davon vorliegt, in Gegenwart eines anorganischen Mangan-Derivats mit einem p-Tolylmagnesiumhalogenid zu den gewünschten Verbindungen umsetzt.

11. Verfahren nach Anspruch 10, **dadurch gekennzeichnet, daß** die Reaktion bei einer Temperatur zwischen -10°C und der Rückflußtemperatur durchgeführt wird.

12. Verfahren nach einem der Ansprüche 10 oder 11, **dadurch gekennzeichnet, daß** die Reaktion in einem aliphatischen oder alicyclischen Ether durchgeführt wird.

13. Verfahren nach einem der Ansprüche 10 oder 11, **dadurch gekennzeichnet, daß** der Ether Tetrahydrofuran, Methyl-tert.-butylether, Dibutylether oder Dioxan ist.

14. Verfahren nach einem der Ansprüche 10 bis 13, **dadurch gekennzeichnet, daß** man 1 bis 2 Moläquivalente p-Tolylmagnesiumchlorid oder -bromid pro Moläquivalent des Benzaldimin-Derivats der Formel III verwendet.

15. Verfahren nach einem der Ansprüche 10 bis 14, **dadurch gekennzeichnet, daß** das anorganische Mangan-Derivat ein Mangansalz oder ein Manganoxid ist.

16. Verfahren nach Anspruch 15, **dadurch gekennzeichnet, daß** das Mangansalz ein Mangan(II)-salz ist und das Manganoxid Mangan(II)-oxid ist.

17. Verfahren nach Anspruch 16, **dadurch gekennzeichnet, daß** das Mangan(II)-salz MnCl₂ oder Li₂MnCl₄ ist.

18. Verfahren nach einem der Ansprüche 10 bis 17, **dadurch gekennzeichnet, daß** das anorganische Mangan-Derivat in einer Menge von 0,1 bis 0,5 Moläquivalenten pro Moläquivalent des Benzaldimin-Derivats der Formel III eingesetzt wird.

19. Verfahren zur Herstellung von o-(p-Tolyl)-benzonitril, **dadurch gekennzeichnet, daß** man das Methyl-biphenyl-Derivat nach Anspruch 2 mit einem Entwässerungsmittel behandelt.

20. Verfahren nach Anspruch 19, **dadurch gekennzeichnet, daß** die Reaktion bei einer Temperatur zwischen der Raumtemperatur und der Rückflußtemperatur des Mediums durchgeführt wird.

21. Verfahren nach Anspruch 19 oder 20, **dadurch gekennzeichnet, daß** die Reaktion in einem aprotischen Lösungsmittel durchgeführt wird.

22. Verfahren nach Anspruch 21, **dadurch gekennzeichnet, daß** das aprotische Lösungsmittel ein Ether ist.

23. Verfahren nach einem der Ansprüche 19 bis 22, **dadurch gekennzeichnet, daß** das Entwässerungsmittel Ameisensäure, Phosphorsäureanhydrid, Phosphoroxidchlorid, Pyridin oder Dicyclohexylcarbodiimid ist.

24. Verfahren zur Herstellung von o-(p-Tolyl)-benzonitril nach Anspruch 19, **dadurch gekennzeichnet, daß** man ein Benzaldimin-Derivat der Formel II nach Anspruch 1:
entweder:
(a) durch Umsetzen mit einem Hydroxylaminsalz in einem aprotischen Lösungsmittel und bei einer Temperatur zwischen 0°C und 10°C in der Weise umwandelt, daß sich als Zwischenprodukt o-(p-Tolyl)-benzaldoxim ergibt, welches man ohne Isolierung bei einer Temperatur zwischen Raumtemperatur und der Rückflußtemperatur zur Bildung der gewünschten Verbindung mit einem Entwässerungsmittel behandelt,
oder:
(b) durch Umsetzen mit Hydroxylamin-O-sulfonsäure in einem aus Wasser und einem aprotischen Lösungsmittel gebildeten zweiphasigen Medium und bei einer Temperatur zwischen Raumtemperatur und der Rückflußtemperatur umwandelt, so daß man als Zwischenprodukt o-(p-Tolyl)-benzaldoxim in Mischung mit o-(p-Tolyl)-benzonitril erhält, welche Mischung man ohne Isolierung von o-(p-Tolyl)-benzaldoxim bei einer Temperatur zwischen Raumtemperatur und der Rückflußtemperatur zur Bildung der gewünschten Verbindung mit einem Entwässerungsmittel behandelt,
oder:
(c) durch Hydrolyse in einem aprotischen Lösungsmittel umwandelt zur Bildung von o-(p-Tolyl)-benzaldehyd, welchen man mit einem Hydroxylaminsalz bei einer Temperatur zwischen 0°C und 10°C umsetzt, so daß man als Zwischenprodukt o-(p-Tolyl)-benzaldoxim erhält, welches man ohne Isolierung bei einer Temperatur zwischen Raumtemperatur und der Rückflußtemperatur des Mediums zur Bildung der gewünschten Verbindung mit einem Entwässerungsmittel behandelt.

25. Verfahren nach Anspruch 24, **dadurch gekennzeichnet, daß** man das Benzaldimin der Formel II durch Umsetzen eines Benzaldimin-Derivats der allgemeinen Formel: 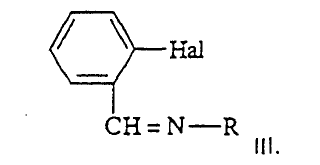 in der R eine geradkettige oder verzweigte C₃-C₇-Alkylgruppe oder eine C₃-C₇-Cycloalkylgruppe bedeutet, welche Verbindung in Form der einzelnen Isomeren oder in Form von Mischungen davon vorliegt, mit einem p-Tolylmagnesiumhalogenid in Gegenwart eines anorganischen Mangan-Derivats und anschließende Umwandlung ohne Isolierung aus seinem Reaktionsmedium erhält.

## Claims

1. Methylbiphenyl derivatives of general formula: 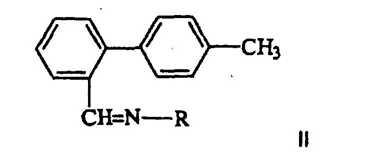 in which R represents a linear or branched C₃-C₇ alkyl group or a C₃-C₇ cycloalkyl group, these compounds being considered in the form of their individual isomers or mixtures thereof.

2. Methylbiphenyl derivative of formula: 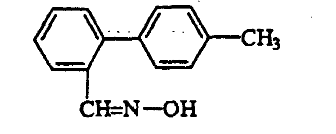 this compound being considered in the form of its individual isomers or mixtures thereof.

3. Methylbiphenyl derivative according to Claim 1, **characterized in that** R represents cyclohexyl.

4. Methylbiphenyl derivative according to Claim 1, **characterized in that** R represents tert-butyl.

5. Process for preparing the methyl-biphenyl derivative according to Claim 2, **characterized in that** a hydroxylamine salt is reacted with a benzaldimine derivative according to one of Claims 1, 3 and 4, to give the desired compound.

6. Process according to Claim 5, **characterized in that** the reaction takes place at a temperature of between 0°C and 10°C.

7. Process according to Claim 5 or 6, **characterized in that** the reaction is carried out in an aprotic solvent.

8. Process according to Claim 7, **characterized in that** the aprotic solvent is an aliphatic or alicyclic ether, an aliphatic or aromatic hydrocarbon or a halogenated hydrocarbon.

9. Process according to Claim 7 or 8, **characterized in that** the aprotic solvent is tetrahydrofuran, methyl tert-butyl ether, dibutyl ether or dioxane.

10. Process for preparing methylbiphenyl derivatives according to one of Claims 1, 3 and 4, **characterized in that** a benzaldimine derivative of general formula: 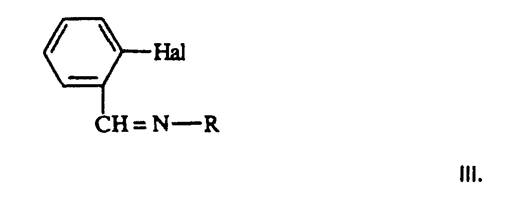 in which R represents a linear or branched C₃-C₇ alkyl group or a C₃-C₇ cycloalkyl group and Hal represents a halogen atom, this compound being in the form of individual isomers or mixtures thereof, is reacted, in the presence of an inorganic manganese derivative, with a p-tolylmagnesium halide, to give the desired compounds.

11. Process according to Claim 10, **characterized in that** the reaction takes place at a temperature of between -10°C and the reflux temperature.

12. Process according to either of Claims 10 and 11, **characterized in that** the reaction takes place in an aliphatic or alicyclic ether.

13. Process according to either of Claims 10 and 11, **characterized in that** the ether is tetrahydrofuran, methyl tert-butyl ether, dibutyl ether or dioxane.

14. Process according to one of Claims 10 to 13, **characterized in that** from 1 to 2 molar equivalents of p-tolylmagnesium chloride or bromide are used per molar equivalent of benzaldimine derivative of formula III.

15. Process according to one of Claims 10 to 14, **characterized in that** the inorganic manganese derivative is a manganese salt or a manganese oxide.

16. Process according to Claim 15, **characterized in that** the manganese salt is a manganous salt and the manganese oxide is manganous oxide.

17. Process according to Claim 16, **characterized in that** the manganous salt is MnCl₂ or MnCl₄Li₂.

18. Process according to one of Claims 10 to 17, **characterized in that** the inorganic manganese derivative is used in a proportion of from 0.1 to 0.5 molar equivalent per molar equivalent of benzaldimine derivative of formula III.

19. Process for preparing o-(p-tolyl)benzonitrile, **characterized in that** the methylbiphenyl derivative according to Claim 2 is subjected to the action of a dehydrating agent.

20. Process according to Claim 19, **characterized in that** the reaction produced is carried out at a temperature of between room temperature and the reflux temperature of the medium.

21. Process according to Claim 19 or 20, **characterized in that** the reaction takes place in an aprotic solvent.

22. Process according to Claim 21, **characterized in that** the aprotic solvent is an ether.

23. Process according to one of Claims 19 to 22, **characterized in that** the dehydrating agent is formic acid, phosphorus pentoxide, phosphorus oxychloride, pyridine or dicyclohexylcarbodiimide.

24. Process for preparing o-(p-tolyl)benzonitrile according to Claim 19, **characterized in that** a benzaldimine derivative of formula II according to Claim 1 is converted:
either:
(a) by reacting this imine in an aprotic solvent with a hydroxylamine salt at a temperature of between 0°C and 10°C, so as to form, transiently and without isolation, o-(p-tolyl)benzaldoxime, which is treated with a dehydrating agent at a temperature of between room temperature and the reflux temperature, giving the desired compound,
or:
(b) by reacting this imine with hydroxylamine-O-sulphonic acid in a two-phase medium formed from water and from an aprotic solvent and at a temperature of between room temperature and the reflux temperature, in order to obtain, transiently and without isolation, o-(p-tolyl)-benzaldoxime as a mixture with o-(p-tolyl)-benzonitrile, this mixture being treated with a dehydrating agent at a temperature of between room temperature and the reflux temperature, giving the desired compound,
or:
(c) by hydrolysing this imine, in an aprotic solvent, to give o-(p-tolyl)benzaldehyde, which is reacted with a hydroxylamine salt at a temperature of between 0°C and 10°C, which gives, transiently and without isolation, o-(p-tolyl)benzaldoxime, which is treated with a dehydrating agent at a temperature of between room temperature and the reflux temperature of the medium, giving the desired compound.

25. Process according to Claim 24, **characterized in that** the benzaldimine derivative of formula II is obtained by reaction between a benzaldimine derivative of general formula: 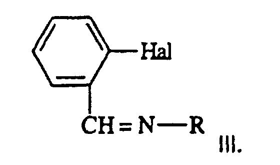 in which R represents a linear or branched C₃-C₇ alkyl group or a C₃-C₇ cycloalkyl group, this compound being in the form of individual isomers or mixtures thereof, with a p-tolylmagnesium halide in the presence of an inorganic manganese derivative, and is then converted without isolation from its reaction medium.
